# EUROPÄISCHE PATENTSCHRIFT

(11) **EP 2 840 975 B1**
(45) Veröffentlichungstag und Bekanntmachung des Hinweises auf die Patenterteilung: **13.06.2018**
(21) Anmeldenummer: 13719080.7
(22) Anmeldetag: 23.04.2013
(51) Int. Cl.: A61B 6/08, A61B 6/00, A61N 5/10

(54) **RÖNTGENQUELLE MIT MODUL UND DETEKTOR FÜR OPTISCHE STRAHLUNG**
X-RAY SOURCE WITH MODULE AND DETECTOR FOR OPTICAL RADIATION
SOURCE DE RAYONS X POURVUE D'UN MODULE ET D'UN DÉTECTEUR POUR RAYONNEMENT OPTIQUE

(30) Priorität: 25.04.2012 DE 102012008812
(43) Veröffentlichungstag der Anmeldung: 04.03.2015
(73) Patentinhaber: Fraunhofer Gesellschaft zur Förderung der angewandten Forschung e.V., 80636 München (DE); Charite - Universitätsmedizin Berlin, 10117 Berlin (DE)
(72) Erfinder: KEEVE, Erwin, 14469 Potsdam (DE); STOPP, Fabian, 15366 Neuenhagen (DE); KÄSEBERG, Marc, 16359 Biesenthal (DE); ENGEL, Sebastian, 48147 Münster (DE); UHLMANN, Eckart, 25368 Kiebitzreihe (DE)
(74) Vertreter: Pfenning, Meinig & Partner mbB
(86) Internationale Anmeldenummer: PCT/EP2013/058395
(87) Internationale Veröffentlichungsnummer: WO 2013/160303

(56) Entgegenhaltungen:
- DE-U1- 29 906 438
- US-A1- 2008 049 896
- US-A1- 2008 181 358
- US-A1- 2009 175 406
- US-A1- 2010 091 949
- US-A1- 2011 317 808

## Beschreibung

Die vorliegende Erfindung betrifft eine Röntgenquelle mit einem unmittelbar an der Röntgenquelle angeordneten Modul und einem Detektor für optische Strahlung in Form einer Stereokamera, wobei der Detektor für optische Strahlung unabhängig von der Röntgenquelle bewegbar ist. Röntgenvorrichtungen dienen der zerstörungsfreien Prüfung von zu untersuchenden Objekten. In der Medizin werden Röntgenvorrichtungen insbesondere zur Untersuchung von Patienten eingesetzt. Eine von einer Röntgenquelle der Röntgenvorrichtung ausgesandte Röntgenstrahlung durchdringt hierbei das zu untersuchende Objekt teilweise, wobei eine Absorption der Röntgenstrahlung abhängig von einem durchstrahlten Material unterschiedlich stark ausfällt. Ein durch das Objekt hindurchtretender Anteil der Röntgenstrahlung wird von einem Detektor als Projektionsbild des durchleuchteten Objekts detektiert. In dem Projektionsbild werden räumliche Informationen überlagert dargestellt, wohingegen eine dreidimensionale Darstellung beispielsweise des Körperinneren eine exakte Reposition von Knochenbrüchen an Gelenken oder eine genaue Positionierung von Implantaten relativ zu kritischen anatomischen Strukturen ermöglicht.

Zur Erzeugung dreidimensionaler Bilddaten mit der Röntgenvorrichtung werden mehrere zweidimensionale Projektionsaufnahmen des Objekts aus verschiedenen Raumrichtungen aufgenommen und anschließend über einen Algorithmus ein gescanntes Volumen rekonstruiert. Dies erfolgt bei medizinischer Anwendung beispielsweise vor einem chirurgischen Eingriff. Während des Eingriffs wird ein Chirurg meist durch ein Navigationssystem unterstützt. Eine Planung sowie Durchführung des Eingriffs erfolgt anhand der präoperativ aufgenommenen Bilddaten und der dreidimensionalen Rekonstruktion, allerdings ist es aufgrund der während einer Operation veränderten anatomischen Strukturen des Patienten, die durch eine veränderte Patientenlage oder den bereits teilweise durchgeführten chirurgischen Eingriff gegeben sind, erforderlich, während der Operation anhand aktueller Bilddaten zu planen und zu navigieren.

Eine aus dem Stand der Technik bekannte Lösung sieht daher vor, in einem Operationssaal ein 3D-Röntgensystem, wie beispielsweise einen Computertomographen oder einen 3D-C-Bogen, sowie ein separates Navigationssystem einzusetzen. Da herkömmliche Bildgebungssysteme den Patienten umschließen, können sie in der Regel nicht am Patienten während des Eingriffs verbleiben, weshalb das Navigationssystem und das Bildgebungssystem als zwei getrennte Systeme im Operationssaal aufgebaut sind. Um einen navigierten Eingriff durchzuführen, werden der Patient und das Röntgensystem mit Lokalisatoren versehen und über eine Kamera des Navigationssystems räumlich lokalisiert. Wird beispielsweise ein 3D-C-Bogen in Kombination mit dem Navigationssystem verwendet, müssen der Patient und der C-Bogen gleichzeitig von der Kamera des Navigationssystems lokalisiert werden. Der C-Bogen wird für einen 3D-Scan zum Patienten gefahren und nach dem 3D-Scan wieder von dem Patienten entfernt. Das manuelle Ausrichten der Kamera ist dabei sehr aufwendig und eine fertige Ausrichtung muss gegebenenfalls korrigiert werden, damit sich der an dem Patienten angebrachte Lokalisator und der Lokalisator des Röntgensystems gleichzeitig in einem Arbeitsbereich der Kamera befinden.

Der vorliegenden Erfindung liegt daher die Aufgabe zugrunde, eine Röntgenquelle und eine Röntgenvorrichtung vorzuschlagen, die die genannten Nachteile vermeidet, mit dem also eine Ausrichtung einer Kamera eines Navigationssystems einfacher einzustellen und zu überprüfen ist.

Diese Aufgabe wird erfindungsgemäß gelöst durch eine Röntgenquelle nach Anspruch 1. Vorteilhafte Ausgestaltungen und Weiterbildungen sind in den abhängigen Ansprüchen beschrieben.

Eine erfindungsgemäße Röntgenquelle, die ausgebildet ist, ein abzubildendes Objekt mit Röntgenstrahlung zu bestrahlen, ist in mehrere definierte Aufnahmepositionen relativ zu dem abzubildenden Objekt bewegbar. Unmittelbar an der Röntgenquelle ist ein Modul angeordnet, das eingerichtet ist, eine Markierung auf das abzubildende Objekt zu projizieren und/oder eine Lage des abzubildenden Objekts und/oder eine Lage der auf das abzubildende Objekt projizierten Markierung zu überprüfen. Das Modul umfasst zur Überprüfung der Lage des abzubildenden Objekts und/oder der Markierung einen Detektor für optische Strahlung.

Durch die Anordnung des Moduls unmittelbar an der Röntgenquelle umfasst ein Arbeitsbereich des Moduls ebenfalls das abzubildende Objekt, sofern die Röntgenquelle auf das abzubildende Objekt gerichtet ist. Das Modul als Bestandteil eines Navigationssystems wird somit mit der Röntgenquelle als Bestandteil eines Bildgebungssystems kombiniert. Somit muss das Navigationssystem nicht separat aufgebaut werden, ein Benutzer der Röntgenquelle hat freien Zugang zu dem abzubildenden Objekt und das Navigationssystem ist im Vergleich zu bekannten Lösungen kleinbauender und somit platzsparender ausgeführt. Eine aufwändige Justierung des Moduls entfällt somit. Durch eine Bewegung der Röntgenquelle in eine der Relativpositionen wird automatisch auch das Modul mitbewegt, sodass auch in diesem Fall kein erneutes Ausrichten erfolgen muss. Durch den Detektor wird eine Möglichkeit der Rückkopplung durch Überprüfen der Lage des abzubildenden Objekts bzw. der Markierung ermöglicht, wodurch eine Nachjustierung der Relativposition der Röntgenquelle möglich wird. Unter dem Begriff "Röntgenstrahlung" soll im Rahmen dieser Schrift eine elektromagnetische Strahlung mit einer Wellenlänge zwischen 0,01 nm und 10 nm verstanden werden, während unter dem Begriff "optische Strahlung" im Rahmen dieser Schrift eine elektromagnetische Strahlung im für Menschen sichtbaren Bereich von zwischen 400 nm und 700 nm Wellenlänge sowie in den daran angrenzenden Bereichen ultravioletter und infraroter Strahlung verstanden werden soll. Der der Detektor für optische Strahlung ist als Bestandteil des Moduls unmittelbar an der Röntgenquelle angeordnet, kann jedoch unabhängig von der Röntgenquelle bewegt werden. Hierdurch kann ein größerer Winkelbereich als Arbeitsbereich von dem Detektor erfasst werden. Ebenso kann eine Lichtquelle, die in dem Modul angeordnet ist und zur Markierung verwendet wird, unabhängig von der Röntgenquelle bewegt werden.

Es ist vorgesehen, dass der Detektor eine Stereokamera umfasst, die eingerichtet ist, mindestens einen an dem abzubildenden Objekt und/oder an einem Instrument angebrachten Tracker als Lokalisator zu detektieren. Durch die Stereokamera können auch räumliche Informationen zur Position der Röntgenquelle zu dem abzubildenden Objekt erhalten werden. Außerdem können somit auch die Instrumente, die an dem abzubildenden Objekt eingesetzt werden, und deren Positionen erfasst und verarbeitet werden.

Der Detektor kann zusätzlich auch einen Oberflächenscanner aufweisen, der eingerichtet ist, eine der Röntgenquelle zugewandte Oberfläche des abzubildenden Objekts abzutasten, Neben der Rekonstruktion des aus Projektionsbildern als Röntgendaten erhaltenen Information wird hierdurch eine zusätzlich Information über die Oberfläche des abzubildenden Objekts erhalten, die eine Planung einer Behandlung des Objekts unterstützt.

Zum Projizieren der Markierung sendet das Modul typischerweise Strahlung im sichtbaren Wellenlängenbereich aus, sodass der Benutzer der Röntgenquelle die Strahlung auch in einfacher Weise wahrnehmen kann. Ferner kann auch der Detektor somit die Strahlung detektieren. Alternativ oder zusätzlich kann das Modul auch Strahlung im infraroten oder im ultravioletten Bereich aussenden.

Das Modul umfasst in einer Ausführungsform einen Laser, da somit eine kohärente Lichtquelle bereitgestellt wird, mit der aufgrund des typischerweise kleinen Strahldurchmessers eine punktgenaue Markierung erfolgen kann. Ferner kann je nach verwendetem Lasertyp eine Vielzahl von Wellenlängen zur Markierung verwendet werden. Eine von dem Laser ausgesandte Laserstrahlung liegt wiederum im Allgemeinen im sichtbaren Bereich, kann aber auch im infraroten oder ultravioletten Bereich liegen.

Die Markierung, die das Modul auf das abzubildende Objekt projiziert, kann eine laserbasierte Zielführungslinie sein, durch die der Benutzer zu einem bestimmten Punkt geführt wird.

Es kann ferner vorgesehen sein, dass das Modul einen Laser-Distanz-Sensor aufweist. Hierdurch kann gezielt ein Abstand zwischen der Röntgenquelle bzw. dem Modul und der Oberfläche des abzubildenden Objekts bestimmt werden und die Planung und/oder die projizierte Markierung entsprechend angepasst und genauer spezifiziert werden.

Der Laser, der Laser-Distanzsensor, die Stereokamera sowie der Oberflächenscanner sind typischerweise an die Röntgenquelle abnehmbar anbringbar und können einzeln oder auch alle gemeinsam gleichzeitig verwendet werden. Ferner sind auch beliebige Kombinationen der genannten Bauteile an der Röntgenquelle anbringbar und gleichzeitig verwendbar. Hierdurch wird eine hohe Variabilität der Röntgenquelle und des Moduls erreicht, die je nach erforderlichem Einsatz entsprechend umgerüstet werden kann.

Die Röntgenquelle kann an einer Haltevörrichtung angeordnet sein. Die Haltevorrichtung umfasst vorzugsweise einen in mindestens drei, vorzugsweise in mindestens vier, besonders vorzugsweise in mindestens fünf, typischerweise jedoch in sechs Freiheitsgraden bewegbaren Arm. Die Freiheitsgrade umfassen hierbei im Allgemeinen maximal drei translatorische und/oder maximal drei rotatorische Freiheitsgrade. Hierdurch wird die Röntgenquelle einerseits sicher gehalten, kann andererseits aber immer noch in beliebige Positionen relativ zu dem abzubildenden Objekt bewegt werden.

Die Röntgenquelle ist typischerweise an einem Ende des Arms befestigt, um maximale Bewegungsfreiheit zu gewährleisten. Der Arm kann ein Roboterarm sein oder diesen umfassen, wobei der Roboterarm über entsprechende Gelenke vorzugsweise automatisiert bewegbar ist, sodass eine Ansteuerung der Armbewegung ohne Eingreifen des Benutzers der Röntgenquelle möglich ist. Sofern der Arm manuell bedienbar ist, kann der Arm einen Kraft-Momenten-Sensor zum Unterstützen der Positionierung umfassen.

Vorzugsweise zeigt die Markierung einen Eintrittspunkt sowie einen Eintrittswinkel für eine Behandlung des Objekts, beispielsweise durch eines der Instrumente, an. Das Modul projiziert die Markierung hierzu unter dem Eintrittswinkel auf die Oberfläche des abzubildenden Objekts, d. h. der Eintrittswinkel ist definiert als Winkel zwischen der Oberfläche des abzubildenden Objekts und der als Strahl von dem Modul ausgehend auf diese auftreffenden Markierung und kann beliebige Werte zwischen 0° und 90°, vorzugsweise zwischen 10° und 80°, besonders vorzugsweise zwischen 30° und 60° annehmen. Das Instrument wird zum Ausrichten auf das abzubildende Objekt auf dem projizierten Eintrittspunkt aufgesetzt und wird solange positioniert, bis die projizierte Markierung auf einem Endstück des Instruments liegt. In dieser Position ist das Instrument unter dem gewünschten Eintrittswinkel auf dem abzubildenden Objekt angeordnet.

Typischerweise ist das abzubildende Objekt ein Patient und die Röntgenquelle wird in einem Operationssaal prä- und/oder intraoperativ von einem Chirurgen eingesetzt. Das Instrument ist in diesem Fall vorzugsweise ein chirurgisches Instrument, das besonders vorzugsweise mit einem Instrumententracker versehen ist. Die Planung umfasst in diesem Fall den Eintrittspunkt des chirurgischen Instruments auf einer Hautoberfläche des Patienten und einer Zielstruktur im Inneren des Patienten.

Eine geeignete Röntgenvorrichtung umfasst vorzugsweise eine Röntgenquelle mit den zuvor beschriebenen Eigenschaften, einen Röntgendetektor zum Detektieren der Röntgenstrahlung als Projektionsbild, eine Auswerteeinheit zum Auswerten des von dem Röntgendetektor aufgenommenen Projektionsbilder, wobei das Auswerten ein Berechnen einer Rekonstruktion eines auf den Projektionsbildern aufgenommenen Zielgebiets umfasst, eine Ausgabeeinheit zum Ausgeben des Projektionsbilds bzw. der Projektionsbilder und/oder der Rekonstruktionen sowie eine Steuereinheit zum Ansteuern der Röntgenquelle, des Röntgendetektors, der Auswerteeinheit und der Ausgabeeinheit. Hierdurch können Projektionsbilder als Röntgenaufnahmen gemacht, weiterverarbeitet und ausgewertet werden und die Überprüfung der Lage und bzw. oder Projizieren der Markierung durchgeführt werden. Das üblicherweise separat vorgesehene Navigationssystem ist somit mit dem System zur dreidimensionalen Bildgebung kombiniert. Die Röntgenvorrichtung umfasst wenigstens die Röntgenquelle mit den zuvor beschriebenen Eigenschaften und den Röntgendetektor zum Detektieren der Röntgenstrahlung als Projektionsbild.

Typischerweise sind die Röntgenquelle und der Röntgendetektor räumlich voneinander getrennt und vorzugsweise nicht mechanisch direkt miteinander verbunden. Hierdurch besteht während oder zwischen Aufnahmen für den Benutzer, beispielsweise den Chirurgen, freier Zugang zu dem abzubildenden Objekt. Außerdem können die Röntgenquelle und der Röntgendetektor derart relativ zueinander angeordnet sein, dass die von der Röntgenquelle ausgesandte Röntgenstrahlung orthogonal oder nicht-orthogonal auf den Röntgendetektor auftrifft.

Vorzugsweise ist die Röntgenquelle unabhängig vom Röntgendetektor bewegbar, typischerweise automatisiert bewegbar. Alternativ oder zusätzlich kann auch der Röntgendetektor unabhängig von der Röntgenquelle bewegbar, vorzugsweise automatisiert bewegbar sein.

Die Ausgabe der Projektionsbilder und/oder der Rekonstruktionen kann eine Darstellung auf einem Bildschirm umfassen. Alternativ oder zusätzlich kann die Ausgabe auch auf einem Drucker erfolgen. Der Benutzer erhält somit einfach und eindeutig wahrnehmbare Informationen über das abzubildende Objekt. Die Rekonstruktionen sind hierbei vorzugsweise dreidimensional.

Die Steuereinheit kann ausgebildet sein, die Bewegung der Röntgenquelle und/oder des Moduls sowie die Position der Markierung und die Aufnahme der Projektionsbilder automatisiert zu steuern. Vorzugsweise erfolgt dies über einen Steuerrechner, der eine Software zum Durchführen der genannten Aufgaben sowie eine Planungssoftware aufweist. Mit Hilfe der Planungssoftware kann eine Bearbeitung des abzubildenden Objekts geplant werden und der Benutzer entsprechend zur Bearbeitung angeleitet werden. Hinweise zu der Bearbeitung werden hierzu besonders vorzugsweise ebenfalls auf der Ausgabeeinheit dargestellt. Typischerweise sind die Steuereinheit und die Auswerteeinheit in einem Gerät kombiniert, um Platz zu sparen. Die Röntgenquelle weist die bereits beschriebene Stereokamera auf, und die Röntgenvorrichtung umfasst mindestens einen an dem abzubildenden Objekt und/oder an einem der Instrumente anzubringenden Tracker, der von der Stereokamera erfasst wird. Hierdurch kann die Lage des abzubildenden Objekts bzw. die Lage des jeweiligen Instruments einfach erfasst werden und zur Planung verwendet werden. Alternativ oder zusätzlich kann die Stereokamera auch die von dem Modul projizierte Lichtquelle nachverfolgen.

Typischerweise erfolgt eine Bestimmung der Positionen der Röntgenquelle und des Röntgendetektors sowie des abzubildenden Objekts in einem Basiskoordinatensystem, dessen Ursprung vorzugsweise durch das abzubildende Objekt definiert ist. Durch das Basiskoordinatensystem sind die Positionen der einzelnen Bauteile klar definiert und einfach zu transformieren.

Ein Verfahren, durch das mittels der Röntgenvorrichtung mit den beschriebenen Eigenschaften das abzubildende Objekt überprüft und/oder markiert wird, umfasst mehrere Schritte. In einem Schritt werden mindestens zwei Projektionsbilder des abzubildenden Objekts aus unterschiedlichen Richtungen aufgenommen. Zum Aufnehmen wird das abzubildende Objekt zumindest teilweise von Röntgenstrahlung durchstrahlt, die, je nach Zusammensetzung von in dem abzubildenden Objekt enthaltenen Materialien unterschiedlich stark absorbiert wird. Nicht vollständig absorbierte Anteile der Röntgenstrahlung durchlaufen das abzubildende Objekt und werden mit ihrer jeweiligen Intensität von dem Röntgendetektor detektiert.

Aus den Aufnahmen wird in einem weiteren Schritt eine dreidimensionale Rekonstruktion des aufgenommenen abzubildenden Objekts errechnet. Schließlich wird ein in der dreidimensionalen Rekonstruktion definiertes Zielgebiet oder das abzubildende Objekt durch das an der Röntgenquelle angeordnete Modul markiert und/oder eine Lage des abzubildenden Objekts und/oder der Markierung überprüft. Hierdurch kann das abzubildende Objekt zuverlässig erfasst und wiedergegeben sowie markiert und überprüft werden, sodass der Benutzer detaillierte Informationen zum weiteren Vorgehen erhält.

Typischerweise erfolgt bereits vor dem Aufnehmen der mindestens zwei Projektionsbilder ein erstes Markieren des abzubildenden Objekts und ein erstes Detektieren der Markierung, sodass die Röntgenquelle anhand der Markierung ausgerichtet werden kann.

Das Rekonstruieren des abzubildenden Objekts erfolgt vorzugsweise durch einen iterativen Algorithmus, sodass die Rekonstruktion des aufgenommenen Objekts mit Projektionsbildern aus beliebigen Richtungen ermöglicht wird.

Anhand der Rekonstruktionen wird typischerweise jeweils der Eintrittspunkt und der Eintrittswinkel auf dem abzubildenden Objekt angezeigt, sodass der Benutzer bei Verwenden der Instrumente angeleitet wird.

Vorzugsweise erfolgt die Überprüfung der Lage des Zielgebiets bereits während der Aufnahme der Projektionsbilder, sodass Lageänderungen bereits früh festgestellt und entsprechende Korrekturen vorgenommen werden können.

Vorzugsweise erfolgt fortlaufend eine Anpassung einer Ausrichtung der Stereokamera zum Nachverfolgen eines der Tracker. Die an dem abzubildenden Objekt bzw. einem der Instrumente angeordneten Tracker sind somit stets in dem Arbeitsbereich der Stereokamera und werden von dieser erfasst.

Besonders vorzugsweise wird nach der Überprüfung der Lage des abzubildenden Objekts in einem Verfahrensschritt in Abhängigkeit von der festgestellten Lage des abzubildenden Objekts die Ausrichtung der Röntgenquelle relativ zu dem abzubildenden Objekt angepasst. Dies erfolgt typischerweise automatisiert.

Außerdem kann auch vorgesehen sein, dass die Aufnahme der Projektionsbilder und das Projizieren der Markierung in Echtzeit erfolgen, sodass der Benutzer der Röntgenvorrichtung sofort eine Rückmeldung für das weitere Verfahren erhält.

Falls Tracker verwendet werden, kann eine Relativposition der Röntgenquelle zu einem der Tracker abgespeichert und zu einem späteren Zeitpunkt erneut angefahren werden. Somit ist eine Aufnahme aus einer gleichen Position auch bei zwischenzeitlicher Bewegung des abzubildenden Objekts möglich.

Statt das abzubildende Objekt komplett aufzunehmen, kann auch nur das Zielgebiet als ein Teil des abzubildenden Objekts abgebildet werden. Hierdurch wird eine unnötige Strahlenexposition des restlichen Objekts vermieden. Das Zielgebiet, das auch als Zielstruktur bezeichnet wird, ist typischerweise in einer der dreidimensionalen Rekonstruktionen definiert.

Falls es sich bei dem abzubildenden Objekt um einen Patienten handelt, kann in einem weiteren Verfahrensschritt, der im Allgemeinen vor dem Beginn der Röntgenaufnahmen durchgeführt wird, eine Patientenregistrierung erfolgen, bei der Daten des Patienten erfasst und mit den im Folgenden aufgenommenen Projektionsbildern verknüpft werden. Die Daten umfassen hierbei Lageinformationen der dreidimensionalen Rekonstruktion sowie Lagedaten, die von der Stereokamera oder des Oberflächenscanners ermittelt werden. Die Patientenregistrierung beschreibt eine Transformation zwischen den 3D-Bilddaten des Patienten und einer aktuellen Lage des Patienten im Raum und ermöglicht eine Zeitersparnis bei der Planung und Durchführung der Behandlung des Objekts.

Vorzugsweise erfolgt als erster Schritt des Verfahrens eine Kalibrierung, bei der Lageinformationen, also Positionen der Röntgenquelle und des Röntgendetektors, in dem Basiskoordinatensystem definiert werden.

Ausführungsbeispiele der Erfindung sind in den Zeichnungen dargestellt und werden nachfolgend anhand der Figuren 1 bis 3 erläutert.

Es zeigen:
- Fig. 1: eine Seitenansicht einer Röntgenvorrichtung mit Röntgenquelle und Röntgendetektor,
- Fig. 2: die in Fig. 1 gezeigte Röntgenvorrichtung mit einem Instrument in einer um 90° gegenüber der Ansicht der Fig. 1 gedrehten Ansicht und
- Fig. 3: ein weiteres Ausführungsbeispiel der in Fig. 1 dargestellten Röntgenvorrichtung.

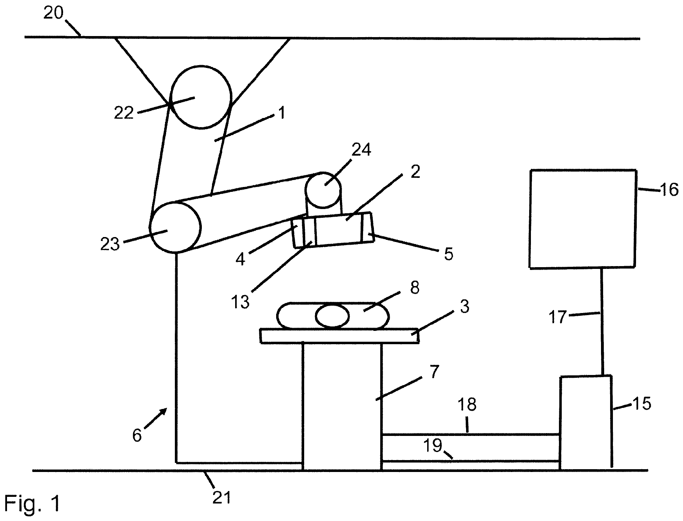

Figur 1 zeigt in einer Seitenansicht eine Röntgenvorrichtung 6 in einem Operationssaal. Die Röntgenvorrichtung 6 ist konzipiert für eine Generierung von Volumendaten eines Patienten 8 als abzubildenden Objekts während eines chirurgischen Eingriffs und für eine sofortige Bereitstellung von Navigationsinformationen basierend auf aktuellen Patientendaten. Medizinische Anwendungsgebiete hierfür sind beispielsweise Unfallchirurgie, Mund-, Kiefer- und Gesichtschirurgie, Orthopädie, Neurologie, Urologie und Kardiologie.

Die Röntgenvorrichtung 6 umfasst eine Röntgenquelle 2, die an einem Ende eines Roboterarms 1 befestigt ist, einen Röntgendetektor 3 sowie einen Rechner 15, der als Auswerteeinheit sowie Steuereinheit dient. Außerdem umfasst die Röntgenvorrichtung 6 einen Bildschirm 16 als Ausgabeeinheit. Der Rechner 15 ist über ein Kabel 17 mit dem Bildschirm 16 verbunden. Außerdem ist der Rechner 15 mit der Röntgenquelle 2 und dem Röntgendetektor 3 jeweils über ein Kabel 18, 19 verbunden. Über das Kabel 18 ist der Rechner 15 auch mit dem Roboterarm 1 verbunden und kann diesen automatisiert bewegen. Statt der Verbindung über die Kabel 17, 18, 19 kann der Rechner 15 auch kabellos mit dem Bildschirm 16, dem Röntgendetektor 3 und der Röntgenquelle 2 verbunden sein. Der Rechner 15 beinhaltet eine Bildaufnahme-, eine Anzeige-, eine 3D-Rekonstruktions-, eine Planungs- und eine Steuersoftware.

Der Roboterarm 1 ist an einer Decke 20 des Operationssaals befestigt, kann in weiteren Ausführungsformen aber natürlich auch auf einem Boden 21 angebracht sein. Der Roboterarm 1 weist drei Gelenke 22, 23, 24 auf, durch die der Roboterarm 1 und somit die an dem Ende des Roboterarms befestigte Röntgenquelle 2 in insgesamt sechs Freiheitsgraden, drei translatorischen Freiheitsgraden und drei rotatorischen Freiheitsgraden, bewegt werden kann. Außerdem umfasst der Roboterarm 1 einen Kraft-Momenten-Sensor, um die Röntgenquelle 2 auch über eine manuelle Krafteinwirkung bzw. eine manuelle Kraftführung verfahren und ausrichten zu können. Durch den Roboterarm 1 ist die Röntgenquelle 2 in mehrere Relativpositionen zu dem Röntgendetektor 3 verfahrbar. Die Relativpositionen können oberhalb, unterhalb, aber auch seitlich des Röntgendetektors 3 liegen. Statt an dem Roboterarm 1 kann die Röntgenquelle 2 auch an einem beispielsweise auf einem Wagen verfahrbaren Stativarm befestigt sein.

Unmittelbar an der Röntgenquelle 2 ist ein Modul befestigt, das eine Stereokamera 5, ein Laser-Licht-Modul 4 mit Laserdistanzsensor und einen Oberflächenscanner 13 umfasst. Das Modul ist abnehmbar anbringbar an die Röntgenquelle 2, wobei die Bauteile des Moduls, also die Stereokamera 5, das Laserlichtmodul 4 und der Oberflächenscanner 13 auch einzeln oder in beliebigen Kombinationen an der Röntgenquelle 2 befestigt sein können, Das Modul liegt bündig an der Röntgenquelle 2 an, sodass sich keine Kanten bilden.

Die Röntgenquelle 2 ist ausgebildet, Röntgenstrahlen auszusenden, die von dem Röntgendetektor 3 detektiert werden. Der Röntgendetektor 3 liegt auf einem Patiententisch 7 auf. Der Patiententisch 7 ist fest mit dem Boden 21 des Operationssaals verbunden. Auf dem Röntgendetektor 3 liegt der Patient 8 als abzubildendes Objekt auf, sodass die Röntgenquelle 2 durch Bewegen des Roboterarms 1 auch in mehrere Relativpositionen zu dem Patienten 8 bewegt wird. Der Röntgendetektor 3 kann in weiteren Ausführungsformen auch abnehmbar anbringbar an den Patiententisch 7 sein, außerdem kann der Röntgendetektor 3 auch unterhalb einer Patientenauflage angeordnet sein, wobei der Patient 8 auf der Patientenauflage aufliegt.

Ebenso kann der Röntgendetektor 3 an einer Positioniervorrichtung angeordnet sein, die eine Bewegung der Röntgendetektors bezüglich des Patienten 8 ermöglicht. Die Positioniervorrichtung kann vorzugsweise mindestens ein abwinkelbares Seitenelement und/oder einen Knickarm aufweisen, um ein verschieben des Detektors an mehrere verschiedene, zumindest nicht alle auf einer geraden liegende Aufnahmepositionen zu ermöglichen, wobei zugleich durch die Positioniervorrichtung eine eindeutige Zuordnung von Röntgenabbildungen zu Positionen des Detektors möglich ist. Bezüglich der Anordnung des Röntgendetektors sowie der Ausgestaltung der Positioniervorrichtung und/oder des Knickarms wird hiermit auf die deutsche Patentanmeldung 10 2012 005 899.3 Bezug genommen.

Die von der Röntgenquelle 2 ausgesandten Röntgenstrahlen durchlaufen den Patienten 8 und werden unterschiedlich stark im Körper des Patienten 8 absorbiert. Ein Anteil der Röntgenstahlen, der nicht vollständig absorbiert wurde, trifft schließlich auf dem Röntgendetektor 3 auf. Der Röntgendetektor 3 ist ein Flachdetektor auf Halbleiterbasis, der ein durch die auftreffende Röntgenstrahlung erhaltenes Bild über das Kabel 18 an den Rechner 15 weiterleitet.

Der Rechner 15 verarbeitet das Bild, typischerweise eine Projektionsaufnahme, und gibt das verarbeitete Bild in Echtzeit auf dem Bildschirm 16 aus. Sofern mehrere Projektionsaufnahmen des Patienten 8 oder auch nur eines Zielgebiets des Patienten 8 wie etwa einzelner Organe vorliegen, führt der Rechner 15 eine dreidimensionale Rekonstruktion des aufgenommenen Gebiets vorzugsweise mit einem iterativen Algorithmus durch und gibt auch diese Rekonstruktion in Echtzeit auf dem Bildschirm 16 wieder. Anhand der Rekonstruktion wird ein Zielgebiet bestimmt und durch das Modul markiert.

Durch eine Kalibrierung sind Lageinformationen der Röntgenquelle 2 und des Röntgendetektors 3 in einem Basiskoordinatensystem bekannt. Das Basiskoordinatensystem ist ein orthogonales Koordinatensystem, dessen Ursprung im Schwerpunkt des Patienten 8 liegt. Alternativ kann der Ursprung auch in einer Ecke des Röntgendetektors 3 oder in der Basis des Roboterarms liegen. Vor der Aufnahme der Projektionsaufnahmen wurde eine Kalibrierung durchgeführt, bei der Positionen der Röntgenquelle 2 und des Röntgendetektors 3 in dem Basiskoordinatensystem bestimmt wurden. Durch Bewegen des Roboterarms 1 wird die Röntgenquelle 2 in verschiedene Relativpositionen zu dem Patienten 8 bewegt, sodass Röntgenaufnahmen unter verschiedenen Winkeln möglich sind. Die dreidimensionale Rekonstruktion kann dann aufgrund der im Basiskoordinatensystem bekannten Lageinformationen der Röntgenquelle 2 und des Röntgendetektors 3 erfolgen. Die Lage des Zielgebiets als rekonstruiertes Volumen ist schließlich auch im Basiskoordinatensystem bekannt.

Die für den Eingriff benötigten Informationen für die Navigation eines Chirurgen am Patienten 8 werden somit ohne ein Verfahren des Patiententischs 7, also ohne Bewegen des Patienten 8 nach dem Aufnehmen der Projektionsaufnahmen erhalten.

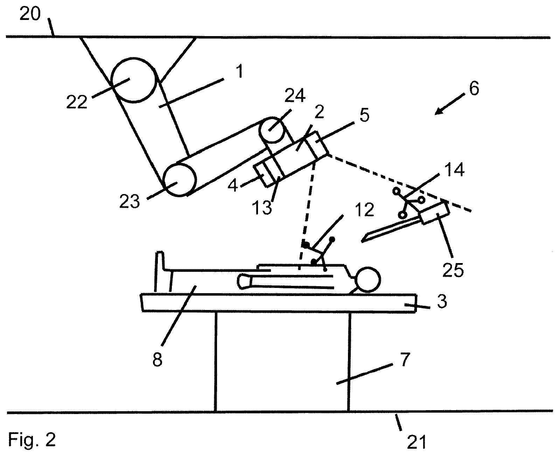

In Fig. 2 ist die Röntgenvorrichtung 6 der Fig. 1 in einer gegenüber der Ansicht der Fig. 1 um 90° gedrehten seitlichen Ansicht dargestellt. Wiederkehrende Bezugszeichen sind in dieser wie auch in den folgenden Figuren mit gleichen Bezugszeichen versehen.

Die Stereokamera 5 ist als Detektor für optische Strahlung (beispielsweise im Wellenlängenbereich zwischen 400 nm und 1mm) ausgelegt und misst die Lage von Trackern 12, 14 in Echtzeit. Die Lage der Stereokamera 5 in dem Basiskoordinatensystem ist durch die Kalibrierung bekannt, allerdings kann die Stereokamera 5 an der Röntgenquelle 2 unabhängig von der Röntgenquelle 2 bewegt werden. Der Patient 8 und etwaige von dem Chirurgen während des Eingriffs benutzte Instrumente sind mit den Trackern 12, 14 als Lokalisatoren versehen. Die Tracker 12, 14 sind aus einer optische Strahlung reflektierenden Struktur gebildet. Vor, während und nach der Aufnahme von Projektionsaufnahmen lokalisiert die Stereokamera 5 einen der Tracker 12, der als Patiententracker an dem Patienten 8 angebracht ist. Hierdurch wird überprüft, ob sich die Lage des Patienten 8 verändert hat. Der weitere Tracker 14 ist als Instrumententracker an dem von dem Chirurgen verwendeten Instrument angebracht und wird ebenfalls von der Stereokamera 5 erfasst.

Anhand der bekannten Lageinformationen der dreidimensionalen Bilddaten und der von der Stereokamera 5 ermittelten Lagedaten des an dem Patienten 8 angebrachten Trackers 12 wird eine automatische Patientenregistrierung durchgeführt. Der Rechner 15 transformiert hierzu die Bilddaten des Patienten 8 und die Lage des Patienten 8 im Basiskoordinatensystem. Durch die Patientenregistrierung kann die Navigationssoftware im Rechner 15 eine Lage der mit Trackern 14 versehenen Instrumente 25 des Chirurgen automatisch in den aufgenommenen dreidimensionalen Bilddaten des Patienten 8 anzeigen und den Chirurgen beim Eingriff durch Anzeige auf dem Bildschirm 16 visuell unterstützen.

Die Stereokamera 5 und das getrackte Instrument 25, das in dem in Fig. 2 gezeigten Ausführungsbeispiel ein Zeigerinstrument ist, werden zur Festlegung des Zielgebiets für eine (gegebenenfalls weitere) dreidimensionale Bilddatenaufnahme benutzt. Eine Festlegung des Zielgebiets durch das getrackte Instrument 25 kann mit oder ohne eine vorhandene Patientenregistrierung erfolgen. Der Chirurg tastet eine anatomische Struktur im Situs des Patienten 8 mit dem getrackten Zeigerinstrument 25 an. Eine Position einer Zeigerspitze des Instruments 25 wird mit der Stereokamera 5 vermessen. Anschließend wird die Bilddatenaufnahme derart durchgeführt, dass das angetastete Zielgebiet im Zentrum eines gescannten Volumens und daher auch im Zentrum der generierten Rekonstruktionen als 3D-Röntgenbilddaten befindet. Darüber hinaus kann auch ohne aufwändige Ausrichtung des Bildgebungssystems eine Relativlage zur angetasteten anatomischen Struktur angegeben werden.

Zur optimalen Ausrichtung der Stereokamera 5 wird diese automatisiert verfahren. Hierdurch wird ein Arbeitsbereich der Stereokamera 5 optimal ausgenutzt bzw. ein Arbeitsbereich des Chirurgen optimal abgebildet. Möglich ist es auch, die Stereokamera 5 auf einen bestimmten der Tracker 12, 14 auszurichten und diesen gegebenenfalls auch während seiner Bewegung nachzuverfolgen und hierbei die Ausrichtung der Stereokamera 5 fortlaufend anzupassen. Durch eine räumliche Trennung der Röntgenquelle 2 und des Röntgendetektors 3 hat der Chirurg während des Eingriffs freien Zugang zu dem Patienten 8 und kann somit auch das Instrument und den daran angebrachten Instrumententracker 14 frei bewegen. Die von der Röntgenquelle 2 kegelförmig ausgesandte Röntgenstrahlung trifft unter beliebigen Winkeln auf den Röntgendetektor 3, wobei ein Kegelzentralstrahl typischerweise orthogonal auf den Röntgendetektor 3 auftrifft.

Nachdem die Stereokamera 5 einmal während der Aufnahme einer zweidimensionalen Projektionsaufnahme zum Patienten 8 ausgerichtet wurde, kann diese Relativlage der Röntgenquelle 2 bezüglich des Patienten 8 und des Röntgendetektors 3 im Basiskoordinatensystem gespeichert werden. Soll zu einem späteren Zeitpunkt einer Operation eine weitere Aufnahme aus dieser Position gemacht werden, bringt der Roboterarm 1 die Röntgenquelle 2 automatisch in die gleiche Stellung bezüglich des am Patienten 8 angebrachten Trackers 14. Der Roboterarm 1 wird hierbei durch den Rechner 15 gesteuert, in dem die Position gespeichert ist. Hierdurch kann trotz eventuell zwischenzeitlich erfolgter Bewegung des Patienten 8 die ursprüngliche Aufnahme wiederholt werden, ohne dass eine manuelle Neuausrichtung der Röntgenvorrichtung 6 vorgenommen werden muss.

Bei einer Verdeckung der Tracker 12, 14 während des chirurgischen Eingriffs, beispielsweise durch weitere notwendige Instrumente, kann über den Roboterarm 1, der von dem Rechner 15 gesteuert wird, die Ausrichtung der Stereokamera 5 optimiert werden, um verbesserte Sichtverhältnisse zwischen der Stereokamera 5 und den Trackern 12, 14 herzustellen. Ortsinformationen der Tracker 12, 14 vor der Verdeckung werden hierzu in Relation gesetzt zu Oberflächendaten während der Verdeckung, die mit dem Oberflächenscanner 13 erhalten werden. Der Oberflächenscanner 13 ist hierfür unabhängig von der Röntgenquelle 2 automatisiert bewegbar. Die Bewegung des Oberflächenscanners 13 wird ebenso wie die Bildaufnahme durch die Röntgenquelle 2 und die Bewegung der Röntgenquelle 2 durch den Rechner 15 gesteuert. Durch die Steuereinheit wird auch das Laser-Licht-Modul 4 angesteuert und der Laserstrahl 11 ein- und ausgeblendet. Anhand der Lageinformationen vor der Verdeckung und Tiefeninformationen bei der Verdeckung wird eine geeignete Position und Ausrichtung der Stereokamera 5 ermittelt, aus welcher sie die Tracker 12, 14 direkt aufnehmen kann. Hierzu kann die Stereokamera 5 beispielsweise in ihrer Höhe verstellt werden.

Falls das Modul keine Stereokamera 5 umfasst, kann die Patientenregistrierung auch nur mit Hilfe des Oberflächenscanners 13 erfolgen und während der Operation aktualisiert werden. Beispielsweise wird für Operationen in der Mund-, Kiefer- und Gesichtschirurgie eine Gesichtsoberfläche des Patienten 8 gescannt und in Relation zur aus den aufgenommenen 3D-Bilddaten extrahierten Hautoberfläche gebracht. Der Oberflächenscanner 13 ist allgemein dazu eingerichtet, eine ihm zugewandte Oberfläche des Patienten 8 abzutasten.

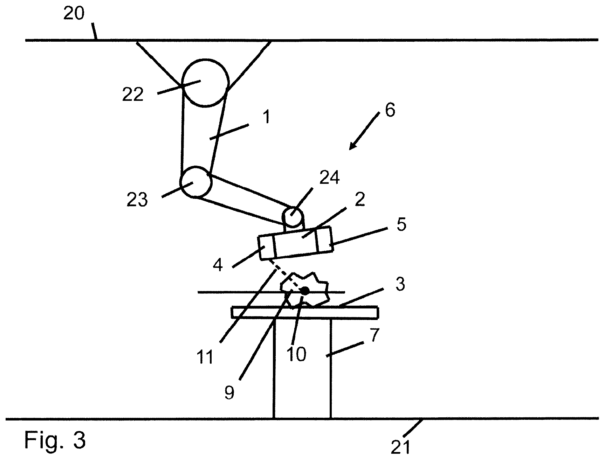

Fig. 3 zeigt in einer Fig. 1 entsprechenden Seitenansicht eine weitere Ausführungsform der Röntgenvorrichtung 6, bei der das Modul, das an der Röntgenquelle 2 angeordnet ist, nur die Stereokamera 5 und das Laser-Licht-Modul 4 umfasst. Das Laser-Licht-Modul 4 umfasst einen Laser als Lichtquelle, der einen Laserstrahl 11 im sichtbaren Wellenlängenbereich emittiert. Durch den Laserstrahl 11 ist ein Laserpunkt oder ein Laserfadenkreuz als Markierung einblendbar. Der Laserstrahl 11 kann jedoch auch als Zielführungslinie dienen, um den Chirurgen zu einem bestimmten zu behandelnden Gebiet zu leiten. Alternativ oder zusätzlich kann das Laser-Licht-Modul 4 auch einen Laserstrahl im infraroten oder ultravioletten Wellenlängenbereich aussenden. Die Markierung kann das Zielgebiet bezeichnen, wobei die Stereokamera 5 die Lage des Patienten 8 und der Markierung überprüft. Bereits vor dem Aufnehmen der ersten Projektionsaufnahmen wird der Laserstrahl 11 als erste Markierung auf dem Patienten 8 eingeblendet, Die Stereokamera 5 überprüft die Lage des Patienten 8 auch während des Aufnehmens der Projektionsaufnahmen. In Abhängigkeit von der von der Stereokamera 5 detektierten Lage des Patienten 8 wird die Ausrichtung der Röntgenquelle 2 relativ zu dem Patienten 8 automatisiert angepasst.

Das Laser-Licht-Modul 4 ist ebenso wie die Stereokamera 5 unabhängig von der Röntgenquelle 2 bewegbar, sodass der Laserstrahl 11 unter unterschiedlichen Winkeln zur Röntgenquelle 2 emittiert wird. Die Lage des Laser-Licht-Moduls 4 und des Laserstrahls 5 sind in dem Basiskoordinatensystem bekannt. Über die in dem Rechner 15 gespeicherte Navigationssoftware kann in den 3D-Bilddaten ein Eintrittspunkt und ein Eintrittswinkel 9 zu einer Zielregion 10 bzw. Zielgebiet des Patienten 8 geplant werden. Der Laserstrahl 11 trifft unter dem Eintrittswinkel 9, der im vorliegenden Ausführungsbeispiel 45° beträgt, auf den Eintrittspunkt auf der Oberfläche des Patienten 8 auf. Die Zielregion 10 befindet sich im Inneren des Patienten 8 und ist in dem in Fig. 3 dargestellten Ausführungsbeispiel die Lunge. Um zu der Zielregion 10 zu gelangen, wird eine Biopsienadel auf den Eintrittspunkt aufgesetzt und solange gedreht, bis der Laserstrahl 11 auf einem Griff der Biopsienadel auftrifft. Die Biopsienadel ist somit an der richtigen Position und weist die richtige Ausrichtung für die Biopsie auf.

Im direkten Anschluss an die 3D-Bilddatenaufnahme, die das Zielgebiet beinhaltet, und nachfolgender Planung richtet der Rechner 15 über den Roboterarm 1 das Laser-Licht-Modul 4 derart aus, dass der Laserstrahl 11 den Eintrittspunkt und den Eintrittswinkel 9 anzeigt. Der Patient 8 wird hierbei zwischen der 3D-Bilddatenaufnahme und der Zielführungsanzeige des Laser-Licht-Moduls 4 nicht bewegt.

Sofern die Röntgenvorrichtung 6 zusätzlich, wie in Fig. 3 dargestellt, mit der Stereokamera 5 versehen ist, kann der Patient 8 zwischen der 3D-Bilddatenaufnahme, der Planung und der Zielführungsanzeige bewegt werden. Der Rechner 15 steuert den Roboterarm 1 dann derart, dass die Bewegung des Patienten 8 durch eine entsprechende Lageänderung des Laser-Licht-Moduls 4 in Bezug zum Tracker 12 automatisch ausgeglichen wird.

Das Laser-Licht-Modul 4 umfasst außerdem den Laserdistanzsensor, der zur Distanzmessung zwischen der Röntgenquelle 2 und dem Patienten 8 verwendet wird, Dies vereinfacht die Navigation und die Ausrichtung. Über die Position und Orientierung des Roboterarms 1 ist in Kombination mit dem Laserdistanzsensor die Raumkoordinate des Punktes, auf den der Laserstrahl 11 fokussiert ist, im Basiskoordinatensystem bestimmbar.

Das Laser-Licht-Modul 4 wird zur Ausrichtung der Röntgenquelle 2 oder der Stereokamera 5 am Roboterarm 1 genutzt. Das Laserfadenkreuz als Markierung wird hierzu zunächst auf das Zielgebiet fokussiert. Zur Ausrichtung der 3D-Bilddatenaufnahme sind gegebenenfalls mehrere Ausrichtungen aus unterschiedlichen Richtungen notwendig. Zusätzlich kann auch eine Relativlage zu der mit dem Laserdistanzsensor fokussierten Struktur angegeben werden, beispielsweise eine Tiefe.

Nach der 3D-Bildaufnahme kann das Laserfadenkreuz bzw. der Laserpunkt auf eine weitere Struktur fokussiert werden. Diese Struktur bzw. dieser Punkt wird in den dreidimensionalen Rekonstruktionen angezeigt, um dem Chirurgen eine Identifikation bzw. Orientierung im Situs zu erleichtern.

Sofern Merkmale lediglich in einzelnen der Ausführungsbeispiele offenbart wurden, ist darin keine Einschränkung auf die Verwendung mit diesen Ausführungsbeispielen zu sehen. Vielmehr können diese Merkmale auch mit anderen Ausführungsbeispielen kombiniert werden und gemeinsam beansprucht werden.

## Patentansprüche

1. Röntgenquelle (2) zum Bestrahlen eines abzubildenden Objekts (8) mit Röntgenstrahlung,
wobei die Röntgenquelle (2) in mehrere definierte Aufnahmepositionen relativ zu dem abzubildenden Objekt (8) bewegbar ist, und wobei
ein Modul (4, 5, 13) unmittelbar an der Röntgenquelle (2) angeordnet ist, das eingerichtet ist,
eine Markierung auf das abzubildende Objekt (8) zu projizieren und
eine Lage des abzubildenden Objekts (8) und/oder der Markierung zu überprüfen, und wobei
das Modul (4, 5, 13) zur Überprüfung der Lage des abzubildenden Objekts (8) und/oder der Markierung einen Detektor (5, 13) für optische Strahlung umfasst, wobei der Detektor (5, 13) eine Stereokamera (5) zur Detektion mindestens eines an dem abzubildenden Objekt (8) und/oder an einem Instrument angebrachten Trackers (12, 14) und/oder der Markierung umfasst, **dadurch gekennzeichnet, dass** der Detektor (5, 13) unabhängig von der Röntgenquelle (2) bewegbar ist.

2. Röntgenquelle (2) nach Anspruch 1, **dadurch gekennzeichnet, dass** der Detektor (5, 13) unabhängig von der Röntgenquelle automatisiert bewegbar ist.

3. Röntgenquelle (2) nach Anspruch 1 oder Anspruch 2, **dadurch gekennzeichnet, dass** der Detektor (5) einen Oberflächenscanner (13) zum Abtasten einer der Röntgenquelle (2) zugewandten Oberfläche des abzubildenden Objekts (8) umfasst.

4. Röntgenquelle (2) nach einem der vorhergehenden Ansprüche, **dadurch gekennzeichnet, dass** das Modul (4, 5, 13) zum Projizieren der Markierung Strahlung im sichtbaren Wellenlängenbereich aussendet.

5. Röntgenquelle (2) nach einem der vorhergehenden Ansprüche, **dadurch gekennzeichnet, dass** das Modul (4, 5, 13) einen Laser umfasst.

6. Röntgenquelle (2) nach Anspruch 4, **dadurch gekennzeichnet, dass** der Laser unabhängig von der Röntgenquelle (2) bewegbar, vorzugsweise automatisiert bewegbar ist.

7. Röntgenquelle (2) nach einem der vorhergehenden Ansprüche, **dadurch gekennzeichnet, dass** das Modul (4, 5, 13) einen Laser-Distanz-Sensor aufweist.

8. Röntgenquelle (2) nach einem der vorhergehenden Ansprüche, **dadurch gekennzeichnet, dass** die Markierung einen Eintrittspunkt und einen Eintrittswinkel (9), unter dem die Markierung auf das abzubildende Objekt (8) auftrifft, für eine Behandlung des abzubildenden Objekts (8) anzeigt.

## Claims

1. An X-ray source (2) for irradiating an object (8) to be imaged with X-ray radiation,
wherein the X-ray source (2) is movable into several defined recording positions relative to the object (9) to be imaged, and wherein
a module (4, 5, 13) is arranged directly on the X-ray source (2) and is configured
to project a marking onto the object (8) to be imaged and
to check a position of the object (8) to be imaged and/or of the marking, and wherein
the module (4, 5, 13) comprises a detector (5, 13) for optical radiation for checking the position of the object (8) to be imaged and/or of the marking, wherein the detector (5, 13) comprises a stereo camera (5) for the detection of at least one tracker (12, 14) which is attached on the object (8) to be imaged and/or on an instrument, and/or of the marking, **characterized in that** the detector (5, 13) is movable independently of the X-ray souce.

2. An X-ray source (2) according to claim 1, **characterised in that** the detector (5, 13) is movable independently of the X-ray source (2), in an automated manner.

3. An X-ray source (2) according to claim 1 or claim 2, **characterised in that** the detector (5) comprises a surface scanner (13) for scanning a surface of the object (8) to be imaged, said surface facing the X-ray source (2).

4. An X-ray source (2) according to one of the preceding claims, **characterised in that** the module (4, 5, 13) emits radiation in the visible wavelength range, for projecting the marking.

5. An X-ray source (2) according to one of the preceding claims, **characterised in that** the module (4, 5, 13) comprises a laser.

6. An X-ray source (2) according to claim 4, **characterised in that** the laser is movable independently of the X-ray source, preferably in an automated manner.

7. An X-ray source (2) according to one of the preceding claims, **characterised in that** the module (4, 5, 13) has a laser distance sensor.

8. An X-ray source (2) according to one of the preceding claims, **characterised in that** the marking indicates an entry point and an entry angle (9), at which the marking hits the object (8) to be imaged, for a treatment of the object (8) to be imaged.

## Revendications

1. Source de rayons X (2) destinée à soumettre à un rayonnement un objet à représenter (8) à l'aide d'un rayonnement X où la source de rayons X (2) peut se déplacer par rapport à l'objet à représenter (8) dans plusieurs positions de prise de vue définies, et dans laquelle un module (4, 5, 13) est disposé immédiatement au niveau de la source de rayons X (2), qui est conçu pour projeter un marquage sur l'objet à représenter (8) et pour vérifier une position de l'objet à représenter (8) et/ou du marquage, et dans laquelle le module (4, 5, 13) comprend pour la vérification de la position de l'objet à représenter (8) et/ou du marquage, un détecteur (5, 13) pour un rayonnement optique, où le détecteur (5, 13) comprend une caméra stéréo (5) pour la détection d'au moins un dispositif traqueur (12, 14) agencé sur l'objet à représenter (8) et/ou sur un instrument, et/ou du marquage, **caractérisée en ce que** le détecteur (5, 13) peut se déplacer de manière indépendante par rapport à la source de rayons X (2).

2. Source de rayons X (2) selon la revendication 1, **caractérisée en ce que** le détecteur (5, 13) peut se déplacer de façon automatisée indépendamment de la source de rayons X.

3. Source de rayons X (2) selon la revendication 1 ou la revendication 2, **caractérisée en ce que** le détecteur (5) comprend un dispositif de balayage de surface (13) pour l'exploration d'une surface de l'objet à représenter (8) orientée vers la source de rayons X (2).

4. Source de rayons X (2) selon l'une des revendications précédentes, **caractérisée en ce que** le module (4, 5, 13), pour la projection du marquage, émet un rayonnement dans le domaine de longueurs d'ondes visible.

5. Source de rayons X (2) selon l'une des revendications précédentes, **caractérisée en ce que** le module (4, 5, 13) comprend un laser.

6. Source de rayons X (2) selon la revendication 4, **caractérisée en ce que** le laser peut être déplacé, de préférence, peut être déplacé de manière automatisée, indépendamment de la source de rayons X (2).

7. Source de rayons X (2) selon l'une des revendications précédentes, **caractérisée en ce que** le module (4, 5, 13) présente un capteur de distance laser.

8. Source de rayons X (2) selon l'une des revendications précédentes, **caractérisée en ce que** le marquage indique un point d'entrée et un angle d'entrée (9), sous lesquels le marquage est présent sur l'objet à représenter (8) pour un traitement de l'objet à représenter (8).
